# EUROPEAN PATENT SPECIFICATION

(11) **EP 4 545 543 B1**
(45) Date of publication and mention of the grant of the patent: **25.11.2026**
(21) Application number: 23826911.2
(22) Date of filing: 29.05.2023
(51) Int. Cl.: C07F 19/00, B01J 31/26, B01J 37/04, C07D 317/36, C07D 317/38, C07F 3/06, C07F 7/10

(54) **IMMOBILIZED ZINC COMPLEX HAVING GUANIDINE LIGAND, METHOD FOR PRODUCING SAME, AND METHOD FOR PRODUCING CYCLIC CARBONATE USING SAME**
IMMOBILISIERTER ZINKKOMPLEX MIT GUANIDINLIGANDEN, VERFAHREN ZUR HERSTELLUNG DAVON UND VERFAHREN ZUR HERSTELLUNG VON CYCLISCHEM CARBONAT DAMIT
COMPLEXE DE ZINC IMMOBILISÉ AYANT UN LIGAND GUANIDINE, SON PROCÉDÉ DE PRODUCTION ET PROCÉDÉ DE PRODUCTION DE CARBONATE CYCLIQUE L'UTILISANT

(30) Priority: 21.06.2022 JP 2022099506; 07.07.2022 JP 2022109626
(43) Date of publication of application: 30.04.2025
(73) Proprietor: SHIN-ETSU CHEMICAL CO., LTD., Tokyo 1000005 (JP)
(72) Inventor: ICHII, Shun, Joetsu-shi, Niigata 942-8601 (JP); TONOMURA, Yoichi, Joetsu-shi, Niigata 942-8601 (JP); KIYOMORI, Ayumu, Joetsu-shi, Niigata 942-8601 (JP)
(74) Representative: Mewburn Ellis LLP
(86) International application number: PCT/JP2023/019878
(87) International publication number: WO 2023/248723

(56) References cited:
- WO-A1-2015/008854
- WO-A1-2015/008854
- WO-A1-2022/113437
- CN-A- 1 696 123
- CN-A- 101 037 431
- JP-A- 2008 296 066
- XIE HAIBO ET AL: "The effective synthesis of propylene carbonate catalyzed by silica-supported hexaalkylguanidinium chloride", NEW JOURNAL OF CHEMISTRY,20080101ROYAL SOCIETY OF CHEMISTRY, GB, vol. 29, no. 9, 1 January 2005 (2005-01-01), Cambridge, pages 1199, XP093118756, ISSN: 1144-0546, DOI: 10.1039/b504822b
- LIU BO ET AL: "Guanidine Hydrochloride/ZnI2 as Heterogeneous Catalyst for Conversion of CO2 and Epoxides to Cyclic Carbonates under Mild Conditions", CATALYSTS, vol. 5, no. 1, 11 February 2015 (2015-02-11), CH, pages 119 - 130, XP093112852, ISSN: 2073-4344, DOI: 10.3390/catal5010119
- XIE HAIBO, DUAN HAIFENG, LI SHENGHAI, ZHANG SUOBO: "The effective synthesis of propylene carbonate catalyzed by silica-supported hexaalkylguanidinium chloride", NEW JOURNAL OF CHEMISTRY, ROYAL SOCIETY OF CHEMISTRY, GB, vol. 29, no. 9, 1 January 2005 (2005-01-01), GB , pages 1199, XP093118756, ISSN: 1144-0546, DOI: 10.1039/b504822b

## Description

### TECHNICAL FIELD

The present invention relates to an immobilized zinc complex having guanidine ligands, a method for synthesizing the same, and a method for synthesizing cyclic carbonates using the immobilized zinc complex.

### BACKGROUND ART

Cyclic carbonate compounds such as ethylene carbonate and propylene carbonate have excellent properties, including high dielectric constants, ease of derivatization and low toxicity, making them useful as, for example, electrolyte solvents for lithium secondary cells, resin plasticizers, and strarting materials for plastics such as polycarbonates and polyhydroxyurethanes.

One method for synthesizing cyclic carbonate compounds is a reaction involving the cycloaddition of an epoxide and carbon dioxide in the presence of a catalyst. Because this method is capable of fixing carbon dioxide and converting it into useful compounds, it represents an important use of carbon dioxide from the standpoint of carbon neutrality, for which there is a strong social demand.

Homogeneous catalysts such as quaternary ammonium salts and alkali metal salts have hitherto been used as catalysts in cycloaddition reactions between epoxides and carbon dioxide.

On the other hand, immobilized catalysts are useful because they have advantages lacking in homogeneous catalysts, such as the ease with which they can be separated from the product by filtration or the like, their ability to be recovered and reused, and their suitability for continuous synthesis. Specifically, using an immobilized catalyst makes it possible to simplify the catalyst removal step, enhance productivity and atom efficiency, and reduce waste products such as solvents used in the reaction and in washing and catalyst residues. Because of such advantages, the use of immobilized catalysts in cycloaddition reactions between epoxides and carbon dioxide has been actively investigated in recent years.

Phosphonium bromide salt catalysts immobilized on silica gel are known as pioneering examples of immobilized catalysts. For example, in Patent Document 1, cyclic carbonate compounds are synthesized from epoxides and carbon dioxide under 10 atm and 90°C conditions using an alkyl triaryl phosphonium bromide immobilized on silica gel as the catalyst. The catalyst in Patent Document 1 can be easily recovered by filtration following reaction completion, and is reusable without a marked accompanying decline in the catalytic activity. In Patent Document 2, propylene carbonate and ethylene carbonate are continuously synthesized in a catalyst-packed reactor by feeding an epoxide and carbon dioxide under 70 atm and 100°C conditions using a tetraalkyl phosphonium bromide salt-immobilized on silica gel as the catalyst.

Non-Patent Document 1 describes that both a homogeneous and a silica-supported hexaalkylguanidinium chloride are effective catalysts for CO, fixation to carbonate without any solvent under mild reaction conditions (4.5 MPa, 120 °C, 4h), the silica-supported hexaalkylguanidinium chloride showed that it could be recycled easily at least 5 further times without any obvious decrease in its catalytic activity, after simple filtration.

Non-Patent Document 2 describes that the combination of guanidine hydrochloride with co-catalyst ZnI₂ proved to be a highly efficient heterogeneous catalyst for the environmentally benign, solvent-free synthesis of cyclic carbonates under mild reaction conditions. The effects of different co-catalysts as well as reaction parameters including catalyst loadings, CO₂ pressure, reaction temperature, and reaction time on the coupling reaction of CO₂ to propylene oxide were investigated. With the molar ratio of guanidine hydrochloride to ZnI₂ at 5:1, excellent yield (94%) and selectivity (=99%) of propylene carbonate were obtained under 100 °C and at 1 MPa for 1.5 h. Additionally, ZnI₂ could be recycled, but because of the washing loss of guanidine hydrochloride, there was a slight decrease in the yield of propylene carbonate. The activity of the catalytic system could be restored by adding additional 20 mol% of fresh guanidine hydrochloride, thus exhibiting recyclability of the ZnlI₂ catalyst.

### PRIOR ART DOCUMENTS

### PATENT DOCUMENTS

Patent Document 1: JP-A 2008-296066
Patent Document 2: WO 2015/008854 A1
Non-Patent Document 1: Xie, Haibo, et al. "The effective synthesis of propylene carbonate catalyzed by silica-supported hexaalkylguanidinium chloride." New Journal of Chemistry 29.9 (2005): 1199-1203.
Non-Patent Document 2: Liu, Bo, et al. "Guanidine hydrochloride/ZnI2 as heterogeneous catalyst for conversion of CO2 and epoxides to cyclic carbonates under mild conditions." Catalysts 5.1 (2015): 119-130.

### SUMMARY OF INVENTION

### TECHNICAL PROBLEM

By using phosphonium bromide salt catalysts immobilized on silica gel, attempts have been made to achieve greater efficiency owing to the above-described advantages of immobilized catalysts, but these methods require harsh reaction conditions. For example, in the case of the immobilized catalysts in Patent Documents 1 and 2, given their low catalytic activity, a high pressure and a high-temperature such as 10 atm and 90°C or 70 atm and 100°C is necessary in order to synthesize a cyclic carbonate compound in a high yield. With methods that thus require high-pressure and high-temperature reaction conditions, not only is it necessary to use a large surplus of carbon dioxide, high energy costs due to heating arise, which is a challenge from the standpoint of sustainable production. In addition, in Patent Document 2, because the catalyst readily deactivates during the reaction, an alkyl bromide such as 2-bromoethanol must be separately added to suppress this, which is undesirable from the standpoint of atom efficiency.

Accordingly, there exists a strong desire for, in the synthesis of cyclic carbonate compounds from epoxides and carbon dioxide, the development of a high-activity immobilized catalyst which enables the reaction to proceed smoothly under mild reaction conditions such as normal pressure and/or room temperature without the addition of additives.

In light of these circumstances, an object of this invention is to provide a zinc complex having guanidine ligands that has been immobilized on an inorganic support, which complex is capable of synthesizing cyclic carbonate compounds in a good yield under mild conditions such as normal pressure and/or room temperature and can be easily recovered and reused following the reaction. Further objects of the invention are to provide a method for synthesizing such an immobilized zinc complex, and a method for synthesizing cyclic carbonate compounds using this immobilized zinc complex.

### SOLUTION TO PROBLEM

The inventors have conducted intensive investigations aimed at achieving the above objects. As a result, they have discovered that by using an immobilized zinc complex having guanidine ligands as the catalyst in a cycloaddition reaction between an epoxide and carbon dioxide, the corresponding cyclic carbonate compound can be synthesized in a high yield and at a high purity under mild reaction conditions such as normal pressure (0.09 to 0.11 MPa) and/or room temperature (1°C to 30°C), and this immobilized zinc complex catalyst can be easily recovered and reused following reaction completion. This discovery ultimately led to the present invention.

Accordingly, the invention provides:
An immobilized zinc complex having guanidine ligands according to claim 1.
immobilized on the inorganic support through covalent bonds with oxygen atoms on a surface of the inorganic support;
Methods for synthesizing the immobilized zinc complex having guanidine ligands according to claims 2 or 3.

A method for synthesizing a cyclic carbonate compound of general formula (6) below according to claim 4.

### ADVANTAGEOUS EFFECTS OF INVENTION

This invention enables an immobilized zinc complex having guanidine ligands to be obtained. By using this immobilized zinc complex having guanidine ligands as the catalyst, cyclic carbonate compounds can be synthesized in a high yield and at high purity under mild conditions such as normal pressure and/or room temperature. Moreover, the zinc complex having guanidine ligands immobilized on an inorganic support that is used as the catalyst can be easily recovered and reused following reaction completion.

### BRIEF DESCRIPTION OF DRAWINGS

[FIG. 1] FIG. 1 is an IR spectrum of the immobilized zinc bromide complex having N',N',N",N"-tetramethylguanidine ligands obtained in Example 1-1.
[FIG. 2] FIG. 2 is an IR spectrum of the N-[3-(trimethoxysilyl)propyl]-N',N',N",N"-tetramethylguanidine used as a starting material in Example 1-1.
[FIG. 3] FIG. 3 is an ¹H-NMR spectrum of the 4,4'-[(2,2-dimethyl-1,3-propanediyl)bis(oxymethylene)]bis(1,3-dioxolan-2-one) obtained in Example 2-6.

### DESCRIPTION OF EMBODIMENTS

The invention is described more fully below.

The immobilized zinc complex having guanidine ligands according to the invention is composed of at least a guanidine-containing alkoxysilane compound of general formula (1) below (referred to below as "Compound (1)"), a zinc halide of general formula (2) below (referred to below as "Compound (2)") and an inorganic support. Silicon atoms on the zinc complex having guanidine ligands, at each of which a nitrogen atom at the guanidine site on Compound (1) is attached to the zinc atom on Compound (2) through a coordinate bond, are immobilized on the inorganic support through covalent bonds with oxygen atoms on a surface of the inorganic support.

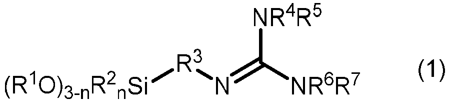

ZnX₂ (2)

In general formula (1) above, R¹ and R² are each independently a substituted or unsubstituted monovalent hydrocarbon group of 1 to 10 carbon atoms, preferably 1 to 5 carbon atoms, and more preferably 1 to 3 carbon atoms.

The monovalent hydrocarbon groups of R¹ and R² may be linear, branched or cyclic. Specific examples include linear alkyl groups such as methyl, ethyl, n-propyl, n-butyl, n-pentyl, n-hexyl, n-heptyl, n-octyl, n-nonyl and n-decyl groups; branched alkyl groups such as isopropyl, isobutyl, sec-butyl, tert-butyl, isopentyl, neopentyl, isohexyl, isoheptyl, isooctyl and tert-octyl groups; cyclic alkyl groups such as cyclopentyl and cyclohexyl groups; alkenyl groups such as vinyl, allyl, 1-propenyl, butenyl and methallyl (2-methyl-2-propenyl) groups; aryl groups such as phenyl, tolyl and xylyl groups; and aralkyl groups such as benzyl and phenethyl groups.

Of these, R¹ and R² are preferably substituted or unsubstituted, linear, branched or cyclic alkyl, alkenyl, aryl or aralkyl groups of 1 to 5 carbon atoms. Particularly from the standpoint of the availability of the starting materials, unsubstituted linear alkyl groups of 1 to 3 carbon atoms are more preferred; methyl and ethyl groups are even more preferred.

Some or all of the hydrogen atoms on these monovalent hydrocarbon groups may be replaced with other substituents selected from the group consisting of alkoxy groups of 1 to 3 carbon atoms, such as methoxy, ethoxy and propoxy groups; halogen atoms such as fluorine, chlorine and bromine; aryl groups of 6 to 10 carbon atoms such as phenyl and tolyl groups; aralkyl groups of 7 to 10 carbon atoms such as benzyl and phenethyl groups; and cyano, amino, ester, ether, carbonyl, acyl and sulfide groups. One or more of these may be used or two or more may be used in combination. No limitations are imposed on the substitution positions of these substituents and on the number of substituents.

In general formula (1), R³ represents an unsubstituted divalent hydrocarbon group of 1 to 10 carbon atoms, preferably 1 to 9 carbon atoms, more preferably 1 to 8 carbon atoms, even more preferably 1 to 5 carbon atoms, and still more preferably 1 to 3 carbon atoms, which may include a heteroatom.

The divalent hydrocarbon group of R³ may be linear, branched or cyclic. Specific examples include alkylene groups such as methylene, ethylene, trimethylene, tetramethylene, isobutylene, hexamethylene, octamethylene, decamethylene, cyclohexylene and methylenecyclohexylene groups; alkenylene groups such as butynylene, propenylene, butenylene, hexenylene and octenylene groups; arylene groups such as the phenylene group; and aralkylene groups such as the methylenephenylene and methylenephenylenemethylene groups.

Of these, R³ is preferably an unsubstituted linear alkylene group of 1 to 8 carbon atoms. Particularly from the standpoint of the availability of the starting materials, methylene, trimethylene and octamethylene groups are more preferred; in terms of the catalytic activity, methylene and trimethylene groups are even more preferred, and the methylene group is still more preferred.

These divalent hydrocarbon groups may have one, two or more intervening heteroatoms on the molecular chain, as in the case of, for example, ether, carbonyl, amino and sulfide groups.

In general formula (1), R⁴ to R⁷ are each independently a hydrogen atom or an unsubstituted monovalent hydrocarbon group of 1 to 10 carbon atoms, preferably 1 to 7 carbon atoms, more preferably 1 to 5 carbon atoms, and even more preferably 1 to 3 carbon atoms.

The monovalent hydrocarbon groups of R⁴ to R⁷ may be linear, branched or cyclic. Specific examples include linear alkyl groups such as methyl, ethyl, n-propyl, n-butyl, n-pentyl, n-hexyl, n-heptyl, n-octyl, n-nonyl and n-decyl groups; branched alkyl groups such as isopropyl, isobutyl, sec-butyl, tert-butyl, thexyl (1, 1,2-trimethylpropyl) and 2-ethylhexyl groups; cyclic alkyl groups such as cyclopentyl and cyclohexyl groups; alkenyl groups such as vinyl, allyl and 1-propenyl groups; aryl groups such as phenyl and tolyl groups; and aralkyl groups such as the benzyl group. Of these, from the standpoint of the availability of the starting materials, methyl, ethyl, isopropyl, cyclohexyl, phenyl and benzyl groups are preferred.

R⁴ and R⁵*,* R⁶ and R⁷, and R⁴ and R⁶ may each mutually bond and form a ring together with the nitrogen atom or atoms to which they are bonded. In cases where a ring is formed, the number of carbons included in the ring is preferably from 3 to 10, more preferably from 3 to 8, and even more preferably from 3 to 6. Examples of such ring structures include the piperidine, pyrrolidine, piperazine, methylpiperazine, tetramethylpiperidine, morpholine, imidazolidine and tetrahydropyrimidine rings.

Specific examples of Compound (1) include N-(alkoxysilyl)alkylguanidines such as N-[3-(trimethoxysilyl)propyl]guanidine, N-[3-(dimethoxymethylsilyl)propyl]guanidine, N-[3-(methoxydimethylsilyl)propyl]guanidine, N-[3-(triethoxysilyl)propyl]guanidine, N-[3-(diethoxymethylsilyl)propyl]guanidine and N-[3-(ethoxydimethylsilyl)propyl]guanidine; N-(alkoxysilyl)alkyl-N'-alkylguanidines such as N-[3-(trimethoxysilyl)propyl]-N'-methylguanidine, N-[3-(dimethoxymethylsilyl)propyl]-N'-methylguanidine, N-[3-(methoxydimethylsilyl)propyl]-N'-methylguanidine, N-[3-(triethoxysilyl)propyl]-N'-methylguanidine, N-[3-(diethoxymethylsilyl)propyl]-N'-methylguanidine, N-[3-(ethoxydimethylsilyl)propyl]-N'-methylguanidine and N-[3-(trimethoxysilyl)propyl]-N'-benzylguanidine; N-(alkoxysilyl)alkyl-N',N'-dialkylguanidines such as N-[3-(trimethoxysilyl)propyl]-N',N'-dimethylguanidine, N-[3-(dimethoxymethylsilyl)propyl]-N',N'-dimethylguanidine, N-[3-(methoxydimethylsily)propyl]-N',N'-dimethylguanidine, N-[3-(triethoxysilyl)propyl]-N',N'-dimethylguanidine, N-[3-(diethoxymethylsilyl)propyl]-N',N'-dimethylguanidine and N-[3-(ethoxydimethylsilyl)propyl]-N',N'-dimethylguanidine; N-(alkoxysilyl)alkyl-N',N"-dialkylguanidines such as N-[3-(trimethoxysilyl)propyl]-N',N"-dimethylguanidine, N-[3-(dimethoxymethylsilyl)propyl]-N',N"-dimethylguanidine, N-[3-(methoxydimethylsilyl)propyl]-N',N"-dimethylguanidine, N-[3-(triethoxysilyl)propyl]-N',N"-dimethylguanidine, N-[3-(diethoxymethylsilyl)propyl]-N',N"-dimethylguanidine, N-[3-(ethoxydimethylsilyl)propyl]-N',N"-dimethylguanidine, N-[3-(trimethoxysilyl)propyl]-N',N"-diethylguanidine, N-[3-(trimethoxysilyl)propyl]-N',N"-diisopropylguanidine and N-[3-(trimethoxysilyl)propyl]-N',N"-dicyclohexylguanidine; N-(alkoxysilyl)alkyl-N',N',N"-trialkylguanidines such as N-[3-(trimethoxysilyl)propyl]-N',N',N"-trimethylguanidine, N-[3-(dimethoxymethylsilyl)propyl]-N',N',N"-trimethylguanidine, N-[3-(methoxydimethylsilyl)propyl]-N',N',N"-trimethylguanidine, N-[3-(triethoxysilyl)propyl]-N',N',N"-trimethylguanidine, N-[3-(diethoxymethylsilyl)propyl]-N',N',N"-trimethyldimethylguanidine and N-[3-(ethoxydimethylsilyl)propyl]-N',N',N"-trimethylguanidine; N-(alkoxysilyl)alkyl-N',N',N",N"-tetraalkylguanidines such as N-[3-(trimethoxysilyl)propyl]-N',N',N",N"-tetramethylguanidine, N-[3-(dimethoxymethylsilyl)propyl]-N',N',N",N"-tetramethylguanidine, N-[3-(methoxydimethylsilyl)propyl]-N',N',N",N"-tetramethylguanidine, N-[3-(triethoxysilyl)propyl]-N',N',N",N"-tetramethylguanidine, N-[3-(diethoxymethylsilyl)propyl]-N',N',N",N"-tetramethylguanidine and N-[3-(ethoxydimethylsilyl)propyl]-N',N',N",N"-tetramethylguanidine; N-(alkoxysilyl)alkylarylguanidines such as N-[3-(trimethoxysilyl)propyl]-N',N"-diphenylguanidine and N-[3-(trimethoxysilyl)propyl]-N'-phenylguanidine; and N-(alkoxysilyl)alkyl cyclic alkyl guanidines such as N-(1,3-dimethyl-2-imidazolidinylidene)-3-(trimethoxysilyl)-1-propanamine and N-(tetrahydro-1,3-dimethyl-2(1H)-pyrimidinylidene)-3-(trimethoxysilyl)-1-propanamine.

Of these, from the standpoint of the availability of the starting materials, N-(alkoxysilyl)alkyl-N',N',N",N"-tetraalkylguanidines are preferred; N-[(trialkoxysilyl)propyl]-N',N',N",N"-tetramethylguanidines and N-[(dialkoxymethylsilyl)propyl]-N',N',N",N"-tetramethylguanidines are more preferred.

Compound (1) may be a commercially available product, or may be synthesized. In cases where it is synthesized, Compound (1) may be obtained in accordance with a known method, such as one that involves subjecting a haloalkylsilane compound and a guanidine compound to a substitution reaction.

In general formula (2) above, X is a halogen atom such as fluorine, chlorine, bromine or iodine. Specific examples of Compound (2) include zinc fluoride, zinc chloride, zinc bromide and zinc iodide. From the standpoints of both the availability of the starting materials and the catalytic activity, zinc bromide is especially preferred.

A commercially available product may be used as Compound (2).

In the immobilized zinc complex of the invention, a "zinc complex having guanidine ligands" refers to a complex in which nitrogen atoms at guanidine sites from Compound (1) are attached to zinc atoms on Compound (2) through coordinate bonds.

In this invention, it is desirable for coordinate bonding to occur between preferably 1.0 to 4.0 moles, and more preferably 1.0 to 2.0 moles, of guanidine sites from Compound (1) and one mole of zinc atoms on Compound (2) to form a four- to six-coordinate zinc complex.

The compound that coordinates with the zinc atoms in the immobilized zinc complex having guanidine ligands of the invention is not limited only to guanidine from Compound (1); solvent molecules may also be coordinated. Here, examples of solvent molecules include the subsequently mentioned reaction solvents that are used when synthesizing the inventive zinc complex having guanidine ligands immobilized on an inorganic support.

The inorganic support used in this invention may be any material having hydroxyl groups on the surface, such as silica gel, mesoporous silica, alumina, zeolite, titania, ceria, zirconia and magnetite. From the standpoint of the availability of the starting materials, silica gel, mesoporous silica, alumina and zeolite are preferred.

Here, given that the catalytic activity is better at higher loadings, the inorganic support immobilizes silicon atoms on Compound (1) through covalent bonds within a range per mole of the silicon atoms of preferably 0.5 to 10.0 kg (equivalent to 2.0 mmol/g to 0.1 mmol/g), and more preferably 0.5 to 2.0 kg (equivalent to 2.0 mmol/g to 0.5 mmol/g).

Next, methods for synthesizing the immobilized zinc complex having guanidine ligands are described.

The immobilized zinc complex having guanidine ligands according to the invention can be synthesized by the two following methods.

The first method involves mixing together Compound (1) and Compound (2) in the presence of, where necessary, an organic solvent, causing nitrogen atoms at guanidine sites on Compound (1) to attach to zinc atoms in the zinc halide through coordinate bonds to form a zinc complex having guanidine ligands. The resulting zinc complex having guanidine ligands is mixed together with an inorganic support, forming covalent bonds between silicon atoms on the zinc complex having guanidine ligands and oxygen atoms on the surface of the inorganic support, thereby immobilizing the zinc complex on the inorganic support.

In the above step in which a zinc complex having guanidine ligands is formed, examples of solvents which may be optionally used include hydrocarbon solvents such as benzene, toluene and xylene; ether solvents such as diethyl ether, tetrahydrofuran, 2-methyltetrahydrofuran, 4-methyltetrahydropyran and dioxane; ester solvents such as ethyl acetate and butyl acetate; aprotic polar solvents such as acetonitrile, N,N-dimethylformamide and N-methylpyrrolidone; and chlorinated hydrocarbon solvents such as dichloromethane and chloroform. These solvents may be used singly or two or more may be used in admixture.

When a solvent is used in the zinc complex-forming step, the amount thereof is not particularly limited, although an amount in the range of preferably 0.1 to 50 liters, and more preferably 0.5 to 20.0 liters, per mole of Compound (2) is suitable.

In the zinc complex-forming step, although the amount of Compound (1) used is not particularly limited, an amount in the range of preferably 1.0 to 4.0 moles, and more preferably 1.0 to 2.0 moles, per mole of Compound (2) is suitable for the formation of a four- to six-coordinate zinc complex.

No limitation is imposed on the pressure at the time of the reaction, although the reaction is preferably carried out at normal pressure.

The reaction temperature is not particularly limited, but is preferably between 0°C and 100°C, and more preferably between 20°C and 80°C. The reaction time also is not particularly limited, but is preferably from 1 to 40 hours, and more preferably from 1 to 10 hours.

The reaction is preferably carried out in an inert gas atmosphere such as nitrogen or argon.

A solvent may be used even in an immobilization step in which a solution of the above zinc complex having guanidine ligands and an inorganic support are mixed together to form covalent bonds between silicon atoms on the zinc complex having guanidine ligands and oxygen atoms on the surface of the inorganic support and thereby immobilize the zinc complex on the inorganic support. This solvent is exemplified by the same solvents as those which can be used in the above zinc complex-forming step.

When a solvent is used in the immobilization step, the amount thereof, although not particularly limited, is preferably from 1.0 to 50.0 liters, and more preferably from 5.0 to 20.0 liters, per mole of Compound (2).

The amount of inorganic support that may be used in the above immobilization step is not particularly limited, but is preferably in the range of 0.5 to 10.0 kg, and more preferably in the range of 0.5 to 2.0 kg, per mole of Compound (1).

The pressure in the immobilization step is not particularly limited, although this step is preferably carried out at normal pressure.

The reaction temperature, although not particularly limited, is preferably between 20°C and 150°C, and more preferably between 50°C and 120°C. The reaction time also is not particularly limited, but is preferably from 1 to 40 hours, and more preferably from 1 to 10 hours.

The reaction is preferably carried out in an inert gas atmosphere such as nitrogen or argon.

The second method for synthesizing the immobilized zinc complex having guanidine ligands according to the invention involves mixing together Compound (1) and an inorganic support in the presence of, where necessary, an organic solvent, causing covalent bonds to form between oxygen atoms present at the surface of the inorganic support and silicon atoms on the guanidine-containing alkoxysilane compound, and thus giving an organic-inorganic composite material in which the guanidine-containing alkoxysilane compound is immobilized on the inorganic support. This organic-inorganic composite material or, in cases where an organic solvent is used, a suspension of the organic-inorganic composite material, is then mixed together with Compound (2), causing nitrogen atoms at guanidine sites within the organic-inorganic composite material to attach to zinc atoms on Compound (2) through coordinate bonds and thus giving a zinc complex having guanidine ligands.

In the organic-inorganic composite material production step, examples of the solvent that may be used where necessary include hydrocarbon solvents such as benzene, toluene and xylene; ether solvents such as diethyl ether, tetrahydrofuran, 2-methyltetrahydrofuran, 4-methyltetrahydropyran and dioxane; ester solvents such as ethyl acetate and butyl acetate; polar aprotic solvents such as acetonitrile, N,N-dimethylformamide and N-methylpyrrolidone; and chlorinated hydrocarbon solvents such as dichloromethane and chloroform. These solvents may be used singly or two or more may be used in admixture.

When a solvent is used, the amount thereof is not particularly limited, but is preferably from 1.0 to 50.0 liters, and more preferably from 5.0 to 20.0 liters, per mole of Compound (1).

In the organic-inorganic composite material production step, the amount of inorganic support used is not particularly limited, but is preferably from 0.5 to 10 kg, and more preferably from 0.5 to 2.0 kg, per mole of Compound (1).

In the organic-inorganic composite material production step, no limitation is imposed on the pressure, although this step is preferably carried out at normal pressure.

The reaction temperature is not particularly limited, but is preferably between 20°C and 150°C, and more preferably between 50°C and 120°C. The reaction time also is not particularly limited, but is preferably from 1 to 40 hours, and more preferably from 1 to 10 hours.

The reaction is preferably carried out in an inert gas atmosphere such as nitrogen or argon.

A solvent may be used even in the step of forming a zinc complex having guanidine ligands. This solvent is exemplified by the same solvents as those which can be used in the above-described organic-inorganic composite material production step.

When a solvent is used, the amount thereof is not particularly limited, but is preferably from 1.0 to 50.0 liters, and more preferably from 5.0 to 20.0 liters, per mole of Compound (2).

The amount of Compound (2) used in the foregoing step is not particularly limited, although an amount of preferably from 0.25 to 1.0 mole, and more preferably from 0.5 to 1.0 mole, per mole of guanidine from Compound (1) is desirable in order to form a four- to six-coordinate zinc complex.

In the above step, no limitation is imposed on the pressure, although this step is preferably carried out at normal pressure.

The reaction temperature is not particularly limited, but is preferably between 0°C and 100°C, and more preferably between 20°C and 80°C. The reaction time also is not particularly limited, but is preferably from 1 to 40 hours, and more preferably from 1 to 10 hours.

The reaction is preferably carried out in an inert gas atmosphere such as nitrogen or argon.

The immobilized zinc complex having guanidine ligands according to this invention can also be obtained by charging a reaction vessel all at once with Compound (1), Compound (2) and an inorganic support, and carrying out the step of forming a zinc complex having guanidine ligands and the immobilization step at the same time.

The immobilized zinc complex having guanidine ligands that has been obtained by the synthesis methods of the invention may also be used after further purification in accordance with the target quality thereof by such methods of purification as filtration, washing and vacuum drying. To obtain a high-purity immobilized zinc complex having guanidine ligands, purification by filtration, washing and vacuum drying is especially preferred.

Next, a method is described for synthesizing cyclic carbonate compounds of general formula (6) below (referred to below as "Compound (6)") by subjecting an epoxide of general formula (3) below (referred to below as "Compound (3)") and carbon dioxide to a cycloaddition reaction using the immobilized zinc complex having guanidine ligands that has been obtained.

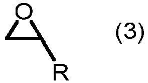

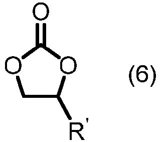

In general formula (3) above, R is a hydrogen atom; a substituted or unsubstituted monovalent hydrocarbon group of 1 to 18 carbon atoms, preferably 1 to 10 carbon atoms, and more preferably 1 to 5 carbon atoms, which may include a heteroatom; a group of general formula (4) below; or a group of general formula (5) below.

-R⁸-SiR⁹ₘ(OR¹⁰)₃₋ₘ (4)

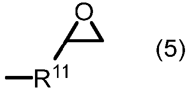

In general formula (3) above, the monovalent hydrocarbon group of R may be linear, branched or cyclic. Specific examples include linear alkyl groups such as methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl and n-octadecyl groups; branched alkyl groups such as isopropyl, isobutyl, sec-butyl, tert-butyl, isopentyl, neopentyl, isohexyl, isoheptyl, isooctyl, tert-octyl, isononyl and isodecyl groups; cyclic alkyl groups such as cyclopentyl and cyclohexyl groups; alkenyl groups such as vinyl, allyl, 1-propenyl, methallyl (2-methyl-2-propenyl) and butenyl groups; aryl groups such as phenyl, tolyl and xylyl groups; and aralkyl groups such as benzyl and phenethyl groups.

Of these, substituted or unsubstituted linear or branched alkyl, alkenyl, aryl and aralkyl groups of 1 to 10 carbon atoms are preferred. Particularly from the standpoint of the availability of the starting materials, alkyl groups of 1 to 6 carbon atoms and aryl groups of 6 to 9 carbon atoms are preferred; methyl, ethyl, butyl and phenyl groups are even more preferred.

Some or all of the hydrogen atoms on these monovalent hydrocarbon groups may be substituted with other substituents. These substituents are the same substituents as for R¹ and R².

Of these, particularly from the standpoint of the availability of the starting materials, such other substituents are more preferably alkoxy groups of 1 to 3 carbon atoms, fluorine atoms, chlorine atoms or phenyl groups.

These monovalent hydrocarbon groups may have one, two or more heteroatoms on the molecular chain, as in the case of, for example, ether, carbonyl, amino and sulfide groups.

In above general formula (4), R⁸ represents an unsubstituted divalent hydrocarbon group of 1 to 10 carbon atoms, preferably 1 to 8 carbon atoms, and more preferably 1 to 5 carbon atoms, which may include a heteroatom. The divalent hydrocarbon group of R⁸ is exemplified by the same substituents as those for R³.

Of these, R⁸ is preferably an unsubstituted linear, ether group-containing, alkylene group of 3 to 10 carbon atoms. Particularly from the standpoint of the availability of the starting materials, the 3-methylene oxide trimethylene group and the 8-methylene oxide octamethylene group are more preferred.

R⁹ and R¹⁰ are each independently a substituted or unsubstituted monovalent hydrocarbon group of 1 to 10 carbon atoms, preferably 1 to 5 carbon atoms, and more preferably 1 to 3 carbon atoms. The monovalent hydrocarbon groups of R⁹ and R¹⁰ are optionally substituted with the same substituents as those for R¹ and R².

Of these, R⁹ and R¹⁰ are more preferably unsubstituted linear alkyl or alkenyl groups of 1 to 3 carbon atoms. Particularly from the standpoint of the availability of the starting materials, methyl and ethyl groups are more preferred.

The letter 'm' is an integer from 0 to 3.

In general formula (5) above, R¹¹ is a substituted or unsubstituted divalent hydrocarbon group of 1 to 20 carbon atoms, preferably 1 to 10 carbon atoms, and more preferably 1 to 5 carbon atoms, which may include a heteroatom.

The divalent hydrocarbon group of R¹¹ may be linear, branched or cyclic. Specific examples include alkylene groups such as methylene, ethylene, trimethylene, tetramethylene, isobutylene, hexamethylene, octamethylene, decamethylene, dodecamethylene, octadecamethylene, cyclohexylene, neopentylene and methylenecyclohexylene groups; alkenylene groups such as butynylene, propenylene, butenylene, hexenylene and octenylene groups; arylene groups such as the phenylene group; and aralkylene groups such as methylenephenylene and methylenephenylenemethylene groups.

Of these, R¹¹ is preferably an unsubstituted alkylene group of 2 to 10 carbon atoms. Particularly from the standpoint of the availability of the starting materials, dimethylene, tetramethylene, methyleneoxydimethyleneoxymethylene and methyleneoxytetramethyleneoxymethylene groups are more preferred.

Some or all of the hydrogen atoms on these divalent hydrocarbon groups may be substituted with other substituents. These substituents are exemplified by the same substituents as in R¹ and R².

Of these, particularly from the standpoint of the availability of the starting materials, alkoxy groups of 1 to 3 carbon atoms, phenyl groups and fluorine atoms are even more preferred as the other substituents.

These divalent hydrocarbon groups may have one, two or more intervening heteroatoms on the molecular chain, as in the case of, for example, ether, carbonyl, amino and sulfide groups.

Specific examples of Compound (3) include aliphatic epoxides such as ethylene oxide, propylene oxide, 1,2-epoxybutane, 1,2-epoxyhexane, 1,2-epoxyoctane and 1,2-epoxydodecane; aromatic epoxides such as styrene oxide; glycidyl ethers such as allyl glycidyl ether, butyl glycidyl ether and benzyl glycidyl ether; silyl group-containing epoxides such as 3-glycidyloxypropyltrimethoxysilane, 3-glycidyloxypropyldimethoxymethylsilane, 3-glycidyloxypropylmethoxydimethylsilane, 3-glycidyloxypropyltriethoxysilane, 3-glycidyloxypropyldiethoxymethylsilane, 3-glycidyloxypropylethoxydimethylsilane, 8-glycidyloxyoctyltrimethoxysilane, 8-glycidyloxyoctyldimethoxymethylsilane, 8-glycidyloxyoctylmethoxydimethylsilane, 8-glycidyloxyoctyltriethoxysilane, 8-glycidyloxyoctyldiethoxymethylsilane and 8-glycidyloxyoctylethoxydimethylsilane; and difunctional epoxides such as 5-hexadiene diepoxide, 1,7-octadiene diepoxide, ethylene glycol diglycidyl ether, 1,4-butanediol diglycidyl ether, 1,6-hexanediol diglycidyl ether, neopentyl glycol diglycidyl ether, 2,2'-bis(4-glycidyloxyphenyl)propane and resorcinol diglycidyl ether.

The amount of carbon dioxide used in the above reaction is not particularly limited. However, from the standpoint of reducing the volume of exhaust gases, the amount of carbon dioxide used per mole of Compound (3) is preferably from 1.0 to 10.0 moles, and more preferably from 1.1 to 3.0 moles.

No limitation is imposed on the carbon dioxide pressure. However, to avoid the need for reaction facilities capable of withstanding pressurization, it is preferable for the reaction to be carried out at a pressure of from 0.09 to 0.11 MPa. Even in cases where pressure is applied in order to shorten the reaction time, the pressure applied is preferably from 0.2 to 10.0 MPa, and more preferably from 0.2 to 8.0 MPa.

The amount in which the zinc complex having guanidine ligands immobilized on an inorganic support is used in the above reaction, expressed in terms of zinc atoms per mole of Compound (3), although not particularly limited, is preferably from 0.001 to 0.1 mole, and more preferably from 0.01 to 0.05 mole.

The above cycloaddition reaction, from the standpoint of reducing energy costs, is preferably carried out at room temperature (between 1°C and 30°C), and more preferably between 10°C and 30°C. Heating may be carried out for the purpose of shortening the reaction time, in which case the reaction temperature is preferably between 40°C and 100°C, and more preferably between 40°C and 70°C. The reaction time, although not particularly limited, is preferably from 1 to 40 hours, and more preferably from 1 to 20 hours.

From the standpoint of environmental compatibility, it is preferable to carry out the cycloaddition reaction in the absence of a solvent. However, a solvent may be used where necessary. Examples of solvents that may be used include hydrocarbon solvents such as benzene, toluene and xylene; ether solvents such as diethyl ether, tetrahydrofuran, 2-methyltetrahydrofuran, 4-methyltetrahydropyran and dioxane; ester solvents such as ethyl acetate and butyl acetate; aprotic polar solvents such as acetonitrile, N,N-dimethylformamide and N-methylpyrrolidone; and chlorinated hydrocarbon solvents such as dichloromethane and chloroform. These solvents may be used singly or two or more may be used in admixture.

In general formula (6) above, R' is a hydrogen atom, a substituted or unsubstituted monovalent hydrocarbon group of 1 to 18 carbon atoms which may include a heteroatom, a group of general formula (4) below, or a group of general formula (7) below.

-R⁸-SiR⁹ₘ(OR¹⁰)₃₋ₘ (4)

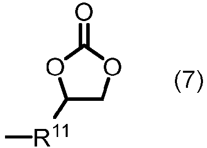

In these formulas, R⁸ to R¹¹ are as defined above.

In general formula (6), the monovalent hydrocarbon group of R' is a substituent similar to cases where R is a substituted or unsubstituted monovalent hydrocarbon group of 1 to 18 carbon atoms which may include a heteroatom.

Specific examples of Compound (6) include aliphatic cyclic carbonates such as ethylene carbonate, propylene carbonate, 4-ethyl-1,3-dioxolan-2-one, 4-butyl-1,3-dioxolan-2-one, 4-hexyl-1,3-dioxolan-2-one and 4-octyl-1,3-dioxolan-2-one; aromatic cyclic carbonates such as 4-phenyl-1,3-dioxolan-2-one; alkoxymethyl cyclic carbonates such as 4-[(2-propen-1-yloxy)methyl]-1,3-dioxolan-2-one, 4-(butoxymethyl)-1,3-dioxolan-2-one and 4-(benzyloxymethyl)-1,3-dioxolan-2-one; silyl group-containing cyclic carbonates such as 4-[(3-trimethoxysilyl)propoxymethyl]-1,3-dioxolan-2-one, 4-[(3-dimethoxymethylsilyl)propoxymethyl]-1,3-dioxolan-2-one, 4-[(3-methoxydimethylsilyl)propoxymethyl]-1,3-dioxolan-2-one, 4-[(3-triethoxysilyl)propoxymethyl]-1,3-dioxolan-2-one, 4-[(3-diethoxymethylsilyl)propoxymethyl]-1,3-dioxolan-2-one, 4-[(3-ethoxydimethylsilyl)propoxymethyl]-1,3-dioxolan-2-one, 4-[(8-trimethoxysilyl)octoxymethyl]-1,3-dioxolan-2-one, 4-[(8-dimethoxymethylsilyl)octoxymethyl]-1,3-dioxolan-2-one, 4-[(8-methoxydimethylsilyl)octoxymethyl]-1,3-dioxolan-2-one, 4-[(8-triethoxysilyl)octoxymethyl]-1,3-dioxolan-2-one, 4-[(8-diethoxymethylsilyl)octoxymethyl]-1,3-dioxolan-2-one and 4-[(8-ethoxydimethylsilyl)octoxymethyl]-1,3-dioxolan-2-one; and difunctional cyclic carbonates such as 4,4'-(1,2-ethanediyl)bis(1,3-dioxolan-2-one), 4,4'-(1,4-butanediyl)bis(1,3-dioxolan-2-one), 4,4'-[(1,2-ethanediylbis(oxymethylene)]bis(1,3-dioxolan-2-one), 4,4'-[(1,4-butanediylbis(oxymethylene)]bis(1,3-dioxolan-2-one), 4,4'-[(1,6-hexanediylbis(oxymethylene)]bis(1,3-dioxolan-2-one), 4,4'-[(2,2-dimethyl-1,3-propanediyl)bis(oxymethylene)]bis(1,3-dioxolan-2-one), 4,4'-[(1-methylethylidene)bis(4,1-phenyleneoxymethylene)]bis(1,3-dioxolan-2-one) and 4,4'-(1,3-phenylenebis(oxymethylene)]bis(1,3-dioxolan-2-one).

The cyclic carbonate compound obtained by the synthesis method of the invention can be further purified in accordance with the target quality by various purification methods such as distillation, filtration, washing and column separation, and then used. To achieve high purity, purification by distillation is especially preferred.

### EXAMPLES

Examples and Comparative Examples are given below by way of illustration, although the invention is not limited by these Examples.

The rate of conversion is expressed as the percent area ratio obtained by gas chromatographic analysis.

### [1] Synthesis of Immobilized Zinc Complexes Having Guanidine Ligands

### [Example 1-1]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 7.9 g (35.0 mmol) of zinc bromide and 150 mL of acetonitrile were charged into the flask and stirred at 25°C. Next, 19.4 g (70.0 mmol) of N-[3-(trimethoxysilyl)propyl]-N',N',N",N"-tetramethylguanidine was added dropwise while adjusting the temperature within the flask to between 25°C and 30°C, following which the flask contents were stirred at 25°C for 2 hours.

The resulting reaction mixture was added to 100.0 g of silica gel (available from Fujifilm Wako Pure Chemical Corporation under the trade name Wakogel^{®} C-200) that had been dried at 120°C and 30 Pa for 2 hours, and then mixed at 80°C for 3 hours. The reaction mixture was cooled to room temperature, after which filtration was carried out through a PTFE membrane having a pore size of 0.2 µm (under a pressure of 0.3 MPa) and the recovered silica gel was washed three times with a mixture of 70 mL of acetone and 30 mL of methanol, giving a treated silica gel. This treated silica gel was dried at 70°C and 30 Pa for 2 hours, giving 125.6 g of an immobilized zinc bromide complex having guanidine ligands.

The resulting immobilized zinc bromide complex having guanidine ligands was confirmed by elemental analysis to have a carbon content of 5.70 wt% and a nitrogen content of 2.21 wt%. The zinc content within the immobilized zinc bromide complex having guanidine ligands, as measured by ICP-OES, was 1.8 wt%. These analytical results indicate that the loadings of guanidine sites in the immobilized zinc bromide complex having guanidine ligands was 0.53 mmol/g and the loadings of zinc atoms was 0.28 mmol/g.

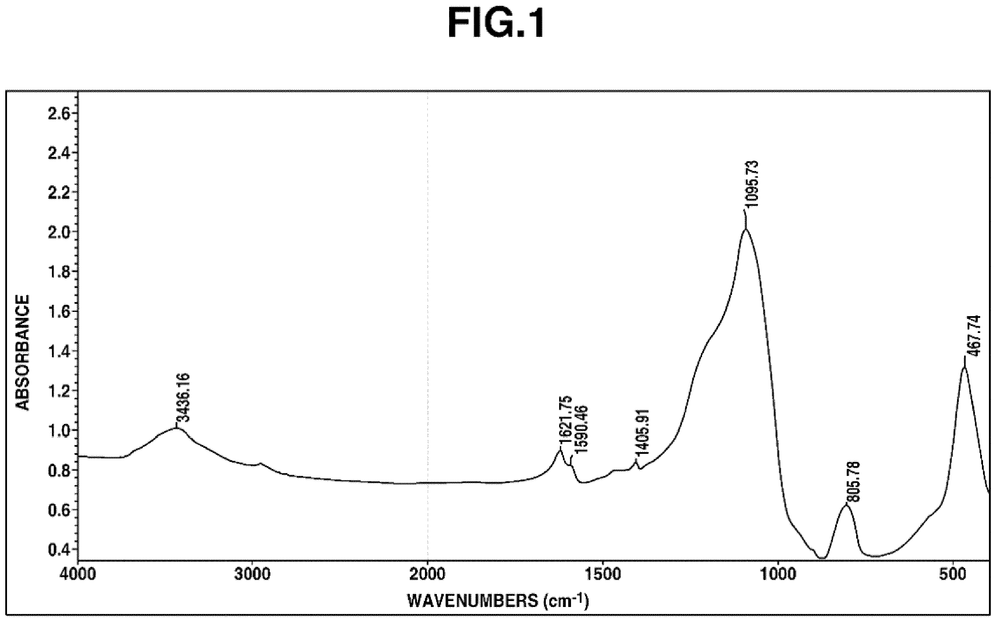

The IR spectrum of the immobilized zinc bromide complex having N',N',N",N"-tetramethylguanidine ligands was measured. The results are shown in FIG. 1.

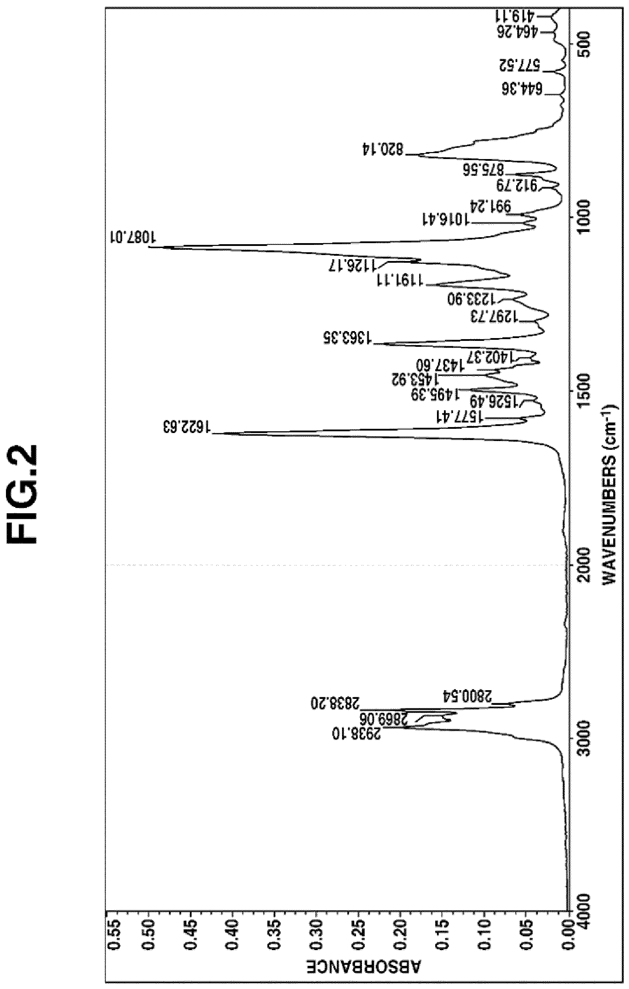

For the sake of comparison, the IR spectrum of the starting material N-[3-(trimethoxysilyl)propyl]-N',N',N",N"-tetramethylguanidine was measured. The results are shown in FIG. 2.

As shown in FIGS. 1 and 2, a characteristic peak is observable at 1590 to 1621 cm⁻¹ in the immobilized zinc bromide complex having N',N',N",N"-tetramethylguanidine ligands, and a peak attributable to the C=N bond at the guanidine sites is observable at 1622 cm⁻¹ in N-[3-(trimethoxysilyl)propyl]-N',N',N",N"-tetramethylguanidine. These results indicate that tetramethylguanidine sites are immobilized on silica gel.

### [Example 1-2]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g of silica gel (available from Fujifilm Wako Pure Chemical Corporation under the trade name Wakogel^{®} C-200) dried at 120°C and 30 Pa for 2 hours and 80 mL of toluene were charged into the flask and stirred at 25°C. Next, 9.7 g (35.0 mmol) of N-[3-(trimethoxysilyl)propyl]-N',N',N",N"-tetramethylguanidine was added dropwise while adjusting the temperature within the flask to between 25°C and 30°C, following which the flask contents were stirred at 110°C for 3 hours. The reaction mixture was cooled to room temperature, after which 40 mL of an acetonitrile solution of 4.0 g (17.5 mmol) of zinc bromide was added while adjusting the temperature within the flask to between 25°C and 30°C and the flask contents were then stirred at 25°C for 1 hour. The reaction mixture was filtered by passing it through a PTFE membrane having a pore size of 0.2 µm (under a pressure of 0.3 MPa), following which the recovered silica gel was washed three times with a mixture of 35 mL of acetone and 15 mL of methanol, giving a treated silica gel. This treated silica gel was dried at 70°C and 30 Pa for 2 hours, giving 62.0 g of an immobilized zinc bromide complex having guanidine ligands.

The resulting immobilized zinc bromide complex having guanidine ligands was confirmed by elemental analysis to have a carbon content of 5.54 wt% and a nitrogen content of 1.98 wt%. The zinc content within the immobilized zinc bromide complex having guanidine ligands, as measured by ICP-OES, was 1.7 wt%. These analytical results indicate that the loadings of guanidine sites in the immobilized zinc bromide complex having guanidine ligands was 0.47 mmol/g and the loadings of zinc atoms was 0.26 mmol/g.

### [Example 1-3]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 79 g (350 mmol) of zinc bromide and 200 mL of acetonitrile were charged into the flask and stirred at 25°C. Next, 194 g (700 mmol) of N-[3-(trimethoxysilyl)propyl]-N',N',N",N"-tetramethylguanidine was added dropwise while adjusting the temperature within the flask to between 25°C and 30°C, following which the flask contents were stirred at 25°C for 2 hours.

The resulting reaction mixture was added to a toluene (1,600 mL) suspension of 1,000.0 g of silica gel (available under the trade name CARiACT^{®} Q-10 from Fuji Silycia Chemical, Ltd.) that had been dried at 120°C and 30 Pa for 2 hours, and stirred at 80°C for 3 hours. The reaction mixture was cooled to room temperature and then filtered by passing it through a SUS filter having a pore size of 800 µm (under a pressure of 0.1 to 0.3 MPa), after which the recovered silica gel was washed three times with a mixture of 700 mL of acetone and 300 mL of methanol, giving a treated silica gel. This treated silica gel was dried at 70°C and 30 Pa for 2 hours, yielding 1,202 g of an immobilized zinc bromide complex having guanidine ligands.

The resulting immobilized zinc bromide complex having guanidine ligands was confirmed by elemental analysis to have a carbon content of 4.99 wt% and a nitrogen content of 1.79 wt%. The zinc content within the immobilized zinc bromide complex having guanidine ligands, as measured by ICP-OES, was 1.7 wt%. These analytical results indicate that the loadings of guanidine sites in the immobilized zinc bromide complex having guanidine ligands was 0.43 mmol/g and the loadings of zinc atoms was 0.26 mmol/g.

### [Example 1-4]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 11.8 g (52.5 mmol) of zinc bromide and 30 mL of acetonitrile were charged into the flask and stirred at 25°C. Next, 30.6 g (105 mmol) of N-[(triethoxysilyl)methyl]-N',N',N",N"-tetramethylguanidine was added dropwise while adjusting the temperature within the flask to between 25°C and 30°C, following which the flask contents were stirred for 2 hours at 25°C.

The resulting reaction mixture was added to a toluene (1,600 mL) suspension of 150.0 g of silica gel (available under the trade name CARiACT^{®} Q-10 from Fuji Silycia Chemical, Ltd.) that had been dried at 120°C and 30 Pa for 2 hours, and stirred at 80°C for 6 hours. The reaction mixture was cooled to room temperature and then filtered by passing it through a SUS filter having a pore size of 800 µm (under a pressure of 0.1 to 0.3 MPa), after which the recovered silica gel was washed three times with a mixture of 110 mL of acetone and 40 mL of methanol, giving a treated silica gel. This treated silica gel was dried at 70°C and 30 Pa for 2 hours, yielding 180.0 g of an immobilized zinc bromide complex having guanidine ligands.

The resulting immobilized zinc bromide complex having guanidine ligands was confirmed by elemental analysis to have a carbon content of 4.34 wt% and a nitrogen content of 1.87 wt%. The zinc content within the immobilized zinc bromide complex having guanidine ligands, as measured by ICP-OES, was 1.4 wt%. These analytical results indicate that the loadings of guanidine sites in the immobilized zinc bromide complex having guanidine ligands was 0.45 mmol/g and the loadings of zinc atoms was 0.21 mmol/g.

### [Example 1-5]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 11.8 g (52.5 mmol) of zinc bromide and 30 mL of acetonitrile were charged into the flask and stirred at 25°C. Next, 40.9 g (105 mmol) of N-[8-(triethoxysilyl)octyl]-N',N',N",N"-tetramethylguanidine was added dropwise while adjusting the temperature within the flask to between 25°C and 30°C, following which the flask contents were stirred at 25°C for 2 hours.

The resulting reaction mixture was added to a toluene (1,600 mL) suspension of 150.0 g of silica gel (available under the trade name CARiACT^{®} Q-10 from Fuji Silycia Chemical, Ltd.) that had been dried at 120°C and 30 Pa for 2 hours, and stirred at 80°C for 6 hours. The reaction mixture was cooled to room temperature and then filtered by passing it through a SUS filter having a pore size of 800 µm (under a pressure of 0.1 to 0.3 MPa), after which the recovered silica gel was washed three times with a mixture of 110 mL of acetone and 40 mL of methanol, giving a treated silica gel. This treated silica gel was dried at 70°C and 30 Pa for 2 hours, yielding 180.6 g of an immobilized zinc bromide complex having guanidine ligands.

The resulting immobilized zinc bromide complex having guanidine ligands was confirmed by elemental analysis to have a carbon content of 6.07 wt% and a nitrogen content of 1.32 wt%. The zinc content within the immobilized zinc bromide complex having guanidine ligands, as measured by ICP-OES, was 1.6 wt%. These analytical results indicate that the loadings of guanidine sites in the immobilized zinc bromide complex having guanidine ligands was 0.31 mmol/g and the loadings of zinc atoms was 0.24 mmol/g.

### [2] Synthesis of Cyclic Carbonate Compounds

### [Example 2-1]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 37.0 g (Zn = 10 mmol) of the immobilized zinc complex having guanidine ligands synthesized in Example 1-1 and 57.1 g (500 mmol) of allyl glycidyl ether were charged into the flask and stirred. Next, while adjusting the temperature to an internal temperature of 27°C, the flask contents were stirred at normal pressure for 20 hours while bubbling carbon dioxide through at a rate of 28 mL/min (amount fed over 20 hours, 1,500 mmol).

A small amount of the reaction mixture was sampled and the conversion rate was determined by gas chromatography, whereupon the rate of conversion from allyl glycidyl ether to 4-[(2-propen-1-yloxy)methyl]-1,3-dioxolan-2-one was 95.0%. Filtration was carried out by passing the reaction mixture through a PTFE membrane having a pore size of 0.2 µm (under a pressure of 0.3 MPa), following which the recovered silica gel was washed three times with 37 mL of toluene. The resulting filtrate was distilled at 0.2 kPa, giving 4-[(2-propen-1-yloxy)methyl]-1,3-dioxolan-2-one at an isolated yield of 87.3% and >99.9% purity.

### [Example 2-2]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g (Zn = 13.50 mmol) of the immobilized zinc complex having guanidine ligands synthesized in Example 1-1, 29.0 g (500 mmol) of propylene oxide and 60 mL of acetonitrile were charged into the flask and stirred. Next, while adjusting the temperature to an internal temperature of 25°C, the flask contents were stirred at normal pressure for 20 hours while bubbling carbon dioxide through at a rate of 28 mL/min (amount fed over 20 hours, 1,500 mmol).

A small amount of the reaction mixture was sampled and the conversion rate was determined by gas chromatography, whereupon the rate of conversion from propylene oxide to propylene carbonate was 95.0%. Filtration was carried out by passing the reaction mixture through a PTFE membrane having a pore size of 0.2 µm (under a pressure of 0.3 MPa), following which the recovered silica gel was washed three times with 50 mL of acetonitrile. The resulting filtrate was distilled at 1.2 kPa, giving propylene carbonate at an isolated yield of 82.5% and >99.9% purity.

### [Example 2-3]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g (Zn = 13.5 mmol) of the immobilized zinc complex having guanidine ligands synthesized in Example 1-1 and 92.0 g (500 mmol) of 2-[(7-octen-1-yloxy)methyl]oxirane were charged into the flask and stirred. Next, while adjusting the temperature to an internal temperature of 70°C, the flask contents were stirred at normal pressure for 8 hours while bubbling carbon dioxide through at a rate of 62.2 mL/min (amount fed over 8 hours, 1,334 mmol).

A small amount of the reaction mixture was sampled and the conversion rate was determined by gas chromatography, whereupon the rate of conversion from 2-[(7-octen-1-yloxy)methyl]oxirane to 4-[(7-octen-1-yloxy)methyl]-1,3-dioxolan-2-one was 96.4%. The reaction mixture was cooled to room temperature and filtered by passing it through a PTFE membrane having a pore size of 0.2 µm (under a pressure of 0.3 MPa), following which the recovered silica gel was washed three times with 50 mL of toluene. The resulting filtrate was distilled at 30 Pa, giving 4-[(7-octen-1-yloxy)methyl]-1,3-dioxolan-2-one at an isolated yield of 90.2% and 98.5% purity.

The reusability as a catalyst of the recovered silica gel which is an immobilized zinc complex having guanidine ligands was confirmed.

Specifically, the recovered silica gel was washed three times with a mixture of 70 mL of acetone and 30 mL of methanol, and then dried at 70°C and 30 Pa for 2 hours. Using this silica gel, the cycloaddition reaction of 2-[(7-octen-1-yloxy)methyl]oxirane was carried out by the same procedure as described above. Following the reaction, the reaction mixture was sampled and the rate of conversion was determined by gas chromatography, whereupon the rate of conversion from 2-[(7-octen-1-yloxy)methyl]oxirane to 4-[(7-octen-1-yloxy)methyl]-1,3-dioxolan-2-one was 94.7%.

### [Example 2-4]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g (Zn = 13.5 mmol) of the immobilized zinc complex having guanidine ligands synthesized in Example 1-1 and 118.0 g (500 mmol) of 3-glycidyloxypropyltrimethoxysilane were charged into the flask and stirred. Next, while adjusting the temperature to an internal temperature of 70°C, the flask contents were stirred at normal pressure for 8 hours while bubbling carbon dioxide through at a rate of 62.2 mL/min (amount fed over 8 hours, 1,334 mmol).

A small amount of the reaction mixture was sampled and the conversion rate was determined by gas chromatography, whereupon the rate of conversion from 3-glycidyloxypropyltrimethoxysilane to 4-[(3-trimethoxysilyl)propoxymethyl]-1,3-dioxolan-2-one was 92.6%. The reaction mixture was cooled to room temperature and filtered by passing it through a PTFE membrane having a pore size of 0.2 µm (under a pressure of 0.3 MPa), following which the recovered silica gel was washed three times with 50 mL of toluene. The resulting filtrate was distilled at 30 Pa, giving 4-[(3-trimethoxysilyl)propoxymethyl]-1,3-dioxolan-2-one at an isolated yield of 80.1% and 99.0% purity.

### [Example 2-5]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g (Zn = 13.5 mmol) of the immobilized zinc complex having guanidine ligands synthesized in Example 1-1 and 153.0 g (500 mmol) of 8-glycidyloxyoctyltrimethoxysilane were charged into the flask and stirred. Next, while adjusting the temperature to an internal temperature of 70°C, the flask contents were stirred at normal pressure for 8 hours while bubbling carbon dioxide through at a rate of 62.2 mL/min (amount fed over 8 hours, 1,334 mmol).

A small amount of the reaction mixture was sampled and the conversion rate was determined by gas chromatography, whereupon the rate of conversion from 8-glycidyloxyoctyltrimethoxysilane to 4-[(8-trimethoxysilyl)octoxymethyl]-1,3-dioxolan-2-one was 91.8%. The reaction mixture was cooled to room temperature and filtered by passing it through a PTFE membrane having a pore size of 0.2 µm (under a pressure of 0.3 MPa), following which the recovered silica gel was washed three times with 50 mL of toluene. The resulting filtrate was distilled at 30 Pa, giving 4-[(8-trimethoxysilyl)octoxymethyl]-1,3-dioxolan-2-one at an isolated yield of 68.5% and 98.9% purity.

### [Example 2-6]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 50.0 g (Zn = 13.5 mmol) of the immobilized zinc complex having guanidine ligands synthesized in Example 1-1 and 108.1 g (500 mmol) of neopentyl glycol diglycidyl ether were charged into the flask and stirred. Next, while adjusting the temperature to an internal temperature of 70°C, the flask contents were stirred at normal pressure for 16 hours while bubbling carbon dioxide through at a rate of 62.2 mL/min (amount fed over 16 hours, 2,668 mmol).

While keeping the reaction mixture warm at 70°C, the reaction mixture was filtered by passing it through a PTFE membrane having a pore size of 0.2 µm (under a pressure of 0.3 MPa), following which the recovered silica gel was washed three times with 50 mL of toluene. The resulting filtrate was concentrated in an evaporator and then dried at 100°C and 30 Pa for one hour, giving 4,4'-[(2,2-dimethyl-1,3-propanediyl)bis(oxymethylene)]bis(1,3-dioxolan-2-one) in a yield of 99.3%.

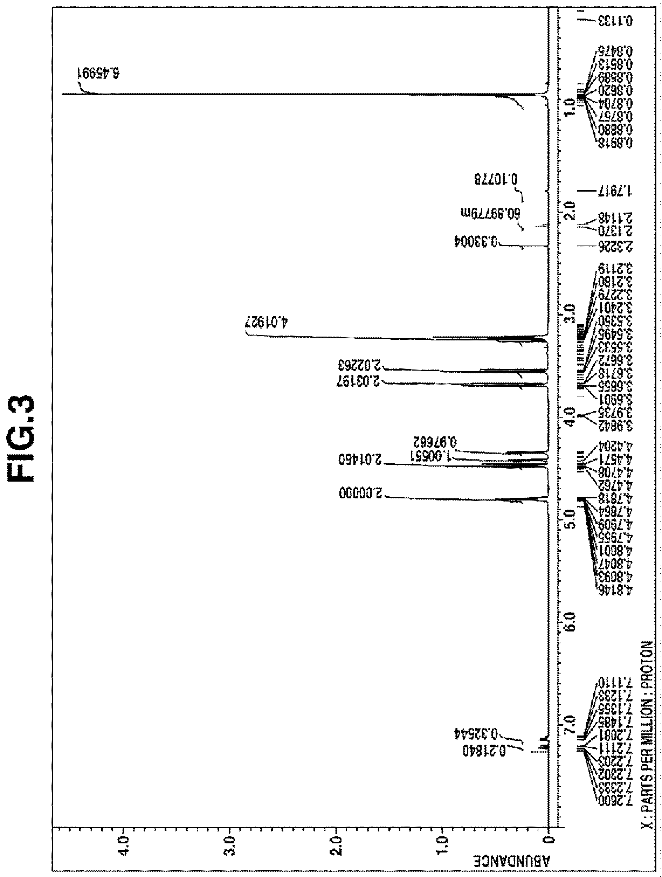

The ¹H-NMR spectrum (heavy chloroform solvent) of the resulting 4,4'-[(2,2-dimethyl-1,3-propanediyl)bis(oxymethylene)]bis(1,3-dioxolan-2-one) was measured. The results are shown in FIG. 3.

As is apparent from FIG. 3, in the ¹H-NMR spectrum, no peaks attributable to the neopentyl glycol diglycidyl ether serving as the starting material or to monocarbonate intermediates in the course of the reaction are observable, confirming that the cycloaddition reaction had reached completion.

Also, substantially no by-product was present in this reaction. In terms of impurities, 0.11 mole of toluene per 1.0 mole of 4,4'-[(2,2-dimethyl-1,3-propanediyl)bis(oxymethylene)]bis(1,3-dioxolan-2-one) was present. Converting this to a weight ratio, the purity of the resulting 4,4'-[(2,2-dimethyl-1,3-propanediyl)bis(oxymethylene)]bis(1,3-dioxolan-2-one) was 96.8%.

### [Example 2-7]

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 13.9 g of the immobilized zinc complex having guanidine ligands synthesized in Example 1-3 and 26.0 g (200 mmol) of butyl glycidyl ether were charged into the flask and stirred. Next, while adjusting the temperature to an internal temperature of 70°C, the flask contents were stirred at normal pressure for 8 hours while bubbling carbon dioxide through at a rate of 24.9 mL/min (amount fed over 8 hours, 534 mmol).

A small amount of the reaction mixture was sampled and the conversion rate was determined by gas chromatography, whereupon the rate of conversion from butyl glycidyl ether to 4-butoxymethyl-1,3-dioxolan-2-one was 94.7%

### [Example 2-8]

Aside from changing the immobilized zinc complex having guanidine ligands synthesized in Example 1-3 to the immobilized zinc complex having guanidine ligands synthesized in Example 1-4, a cycloaddition reaction between butyl glycidyl ether and carbon dioxide was carried out under the same reaction conditions as in Example 2-7.

A small amount of the reaction mixture was sampled and the conversion rate was determined by gas chromatography, whereupon the rate of conversion from butyl glycidyl ether to 4-butoxymethyl-1,3-dioxolan-2-one was 95.2%.

### [Example 2-9]

Aside from changing the immobilized zinc complex having guanidine ligands synthesized in Example 1-3 to the immobilized zinc complex having guanidine ligands synthesized in Example 1-5, a cycloaddition reaction between butyl glycidyl ether and carbon dioxide was carried out under the same reaction conditions as in Example 2-7.

A small amount of the reaction mixture was sampled and the conversion rate was determined by gas chromatography, whereupon the rate of conversion from butyl glycidyl ether to 4-butoxymethyl-1,3-dioxolan-2-one was 90.8%.

### [Comparative Example 1]

A carbonation reaction was carried out on allyl glycidyl ether using the silica gel-immobilized phosphonium bromide salt described in Patent Document 1 and under the same reaction conditions as in Example 2-1. Aside from having the type of silica gel be Wakogel^{®} C-200 from Fujifilm Wako Pure Chemical Corporation, silica gel-immobilized phosphonium bromide derived from tri(p-tolyl)phosphine and 3-bromopropyltriethoxysilane was synthesized in accordance with the method described in Patent Document 1.

The interior of a four-neck flask equipped with a stirrer, a reflux condenser, a dropping funnel and a thermometer was purged with nitrogen and, while passing a stream of nitrogen gas through the open end at the top of the reflux condenser so as to keep outside air from getting in, 37.0 g of the silica gel-immobilized phosphonium bromide salt obtained as described above and 57.1 g (500 mmol) of allyl glycidyl ether were charged into the flask and stirred. Next, while adjusting the temperature to an internal temperature of 27°C, the flask contents were stirred at normal pressure for 20 hours while bubbling carbon dioxide through at a rate of 28 mL/min (amount fed over 20 hours, 2,500 mmol).

A small amount of the reaction mixture was sampled and the conversion rate was determined by gas chromatography, whereupon the rate of conversion from allyl glycidyl ether to 4-[(2-propen-1-yloxy)methyl]-1,3-dioxolan-2-one was 34.9%. The reaction mixture was filtered by passing it through a PTFE membrane having a pore size of 0.2 µm (under a pressure of 0.3 MPa), following which the recovered silica gel was washed three times with 37 mL of toluene. The resulting filtrate was distilled at 0.2 kPa, giving 4-[(2-propen-1-yloxy)methyl]-1,3-dioxolan-2-one at an isolated yield of 20.5% and 99.8% purity.

As demonstrated above, in Examples 2-1 and 2-2, even though the reaction was carried out under mild conditions of normal pressure and room temperature, the corresponding cyclic carbonate compound in both cases was produced at a high rate of conversion and was isolated in a high yield and at high purity. It is apparent from these results that the immobilized zinc complexes having guanidine ligands according to the present invention exhibit excellent catalytic activity even under mild conditions.

In Examples 2-3 to 2-6, it is apparent that when the reaction temperature was set to 70°C in order to shorten the aging time, the cycloaddition reaction proceeded more smoothly and, in each case, the cyclic carbonate compound was obtained in high yield and at a high purity.

In Example 2-3, the reusability as a catalyst of an immobilized zinc complex having guanidine ligands according to the invention was confirmed. As a result, it was found that the rate of conversion of 2-[(7-octen-1-yloxy)methyl]oxirane to 4-[(7-octen-1-yloxy)methyl]-1,3-dioxolan-2-one in the second use of this catalyst was not inferior to that in the first use of the catalyst. This demonstrated that the immobilized zinc complex having guanidine ligands according to this invention can be recovered and reused without substantially lowering the catalytic activity.

In Examples 2-7 to 2-9, the catalytic activities of immobilized zinc complexes in which the guanidine ligand linkers are of differing lengths were compared. When cycloaddition reactions between butyl glycidyl ether and carbon dioxide were carried out under the same reaction conditions, the rate of conversion from butyl glycidyl ether to 4-butoxymethyl-1,3-dioxolan-2-one catalyzed by immobilized zinc complexes having guanidine ligands increased in the following order according to the type of linker: octamethylene linkers (Example 2-9) < trimethylene linkers (Example 2-7) < monomethylene linkers (Example 2-8). That is, it was demonstrated that the guanidine ligands on the immobilized zinc complex according to this invention have a higher catalytic activity when the number of carbon atoms on the linkers is lower.

In Comparative Example 1, using silica gel-immobilized phosphonium bromide salt as the catalyst, a cycloaddition reaction between allyl glycidyl ether and carbon dioxide was carried out at normal pressure and room temperature in the same way as in Example 2-1. As a result, the target substance 4-[(2-propen-1-yloxy)methyl]-1,3-dioxolan-2-one was produced at only a low rate of conversion, and so the isolated yield of the product was exceedingly low.

## Claims

1. An immobilized zinc complex having guanidine ligands, comprising at least a guanidine-containing alkoxysilane compound of general formula (1) below 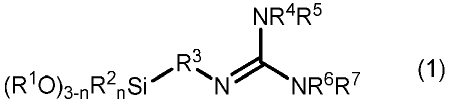
wherein R¹ and R² are each independently a monovalent hydrocarbon group of 1 to 10 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups; R³ is an unsubstituted divalent hydrocarbon group of 1 to 10 carbon atoms which may include a heteroatom; R⁴ to R⁷ are each independently a hydrogen atom or an unsubstituted monovalent hydrocarbon group of 1 to 10 carbon atoms, and R⁴ and R⁵*,* R⁶ and R⁷, and R⁴ and R⁶ may each mutually bond and form a ring together with the nitrogen atom or atoms to which they are bonded; and n is an integer from 0 to 2, a zinc halide of general formula (2) below
ZnX₂ (2)
wherein X is a halogen atom and an inorganic support,
wherein silicon atoms on the zinc complex having guanidine ligands, where nitrogen atoms at sites originating from guanidine on the guanidine-containing alkoxysilane compound are attached to zinc atoms on the zinc halide through coordinate bonds, are immobilized on the inorganic support through covalent bonds with oxygen atoms on a surface of the inorganic support.

2. A method for synthesizing the immobilized zinc complex having guanidine ligands of claim 1, comprising the steps of mixing a guanidine-containing alkoxysilane compound of general formula (1) below 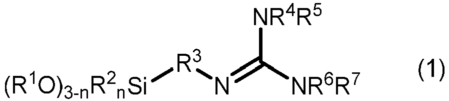
wherein R¹ to R⁷ and n are as defined above with a zinc halide of general formula (2) below
ZnX₂ (2)
wherein X is as defined above, causing nitrogen atoms at guanidine sites on the guanidine-containing alkoxysilane compound to attach to zinc atoms on the zinc halide through coordinate bonds and form a zinc complex having guanidine ligands; and subsequently mixing the resulting zinc complex having guanidine ligands with an inorganic support to form covalent bonds between silicon atoms on the zinc complex having guanidine ligands and oxygen atoms on a surface of the inorganic support and immobilize the zinc complex on the inorganic support.

3. A method for synthesizing the immobilized zinc complex having guanidine ligands of claim 1, comprising the steps of mixing a guanidine-containing alkoxysilane compound of general formula (1) below 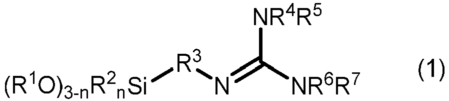
wherein R¹ to R⁷ and n are as defined above with an inorganic support to form covalent bonds between oxygen atoms present on a surface of the inorganic support and silicon atoms on the guanidine-containing alkoxysilane compound and immobilize the guanidine-containing alkoxysilane compound on the inorganic support, producing an organic-inorganic composite material; and subsequently mixing this organic-inorganic composite material with a zinc halide of general formula (2) below
ZnX₂ (2)
wherein X is as defined above, causing nitrogen atoms at guanidine sites included in the organic-inorganic composite material to attach to zinc atoms on the zinc halide through coordinate bonds, producing a zinc complex having guanidine ligands.

4. A method for synthesizing a cyclic carbonate compound of general formula (6) below 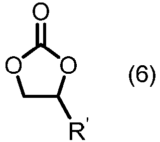
wherein R' is a hydrogen atom, a monovalent hydrocarbon group of 1 to 18 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups, and the monovalent hydrocarbon group may include a heteroatom, a group of general formula (4) below
-R⁸-SiR⁹ₘ(OR¹⁰)₃₋ₘ (4)
wherein R⁸ is an unsubstituted divalent hydrocarbon group of 1 to 10 carbon atoms which may include a heteroatom, R⁹ and R¹⁰ are each independently a monovalent hydrocarbon group of 1 to 10 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups, and m is an integer from 0 to 3 or a group of general formula (7) below 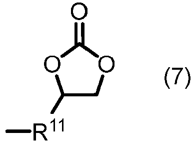
wherein R¹¹ is a divalent hydrocarbon group of 1 to 20 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups, and the divalent hydrocarbon group may include a heteroatom, which method comprises the step of effecting a cycloaddition reaction between an epoxide of general formula (3) below 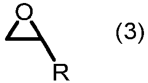
wherein R is a hydrogen atom, a monovalent hydrocarbon group of 1 to 18 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups, and the monovalent hydrocarbon group may include a heteroatom, a group of general formula (4) below
-R⁸-SiR⁹ₘ(OR¹⁰)₃₋ₘ (4)
wherein R⁸ to R¹⁰ and m are as defined above or a group of general formula (5) below 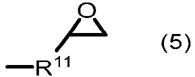
wherein R¹¹ is a divalent hydrocarbon group of 1 to 20 carbon atoms on which some or all of the hydrogen atoms may be replaced with other substituents selected from the group consisting of an alkoxy group of 1 to 3 carbon atoms, a halogen atom, an aryl group of 6 to 10 carbon atoms, an aralkyl groups of 7 to 10 carbon atoms, a cyano group, an amino group, an ester group, an ether group, a carbonyl group, an acyl group and sulfide groups and the divalent hydrocarbon group may include a heteroatom and carbon dioxide in the presence of a catalyst, wherein the immobilized zinc complex having guanidine ligands of claim 1 is used as the catalyst.

## Patentansprüche

1. Immobilisierter Zink-Komplex, der Guanidin-Liganden aufweist und der Folgendes umfasst:
zumindest eine Guanidin-hältige Alkoxysilan-Verbindung der nachstehenden allgemeinen Formel (1): 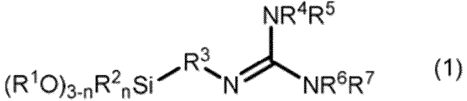
worin R¹ und R² jeweils unabhängig voneinander eine einwertige Kohlenwasserstoffgruppe mit 1 bis 10 Kohlenstoffatomen sind, auf der gegebenenfalls manche oder alle der Wasserstoffatome durch andere Substituenten ersetzt sind, die aus der aus einer Alkoxygruppe mit 1 bis 3 Kohlenstoffatomen, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, R³ eine unsubstituierte zweiwertige Kohlenwasserstoffgruppe mit 1 bis 10 Kohlenstoffatomen ist, die gegebenenfalls ein Heteroatom umfasst, R⁴ bis R⁷ jeweils unabhängig voneinander ein Wasserstoffatom oder eine unsubstituierte einwertige Kohlenwasserstoffgruppe mit 1 bis 10 Kohlenstoffatomen sind und gegebenenfalls R⁴ und R⁵, R⁶ und R⁷ und R⁴ und R⁶ jeweils miteinander verbunden sind und zusammen mit dem Stickstoffatom oder den Atomen, an die sie gebunden sind, einen Ring bilden, und n eine ganze Zahl von 0 bis 2 ist;
ein Zink-Halogenid der nachstehenden allgemeinen Formel (2):
ZnX₂ (2)
worin X ein Halogenatom ist; und
einen anorganischen Träger;
wobei Silicium-Atome auf dem Zink-Komplex, der Guanidin-Liganden aufweist, wo Stickstoff-Atome an Stellen, die von Guanidin auf der Guanidin-hältigen Alkoxysilan-Verbindung herrühren, über koordinative Bindungen an Zink-Atome auf dem Zink-Halogenid gebunden sind, über kovalente Bindungen mit Sauerstoff-Atomen auf einer Oberfläche des anorganischen Trägers auf dem anorganischen Träger immobilisiert sind.

2. Verfahren zum Synthetisieren eines immobilisierten Zink-Komplexes, der Guanidin-Liganden aufweist, nach Anspruch 1, das die Schritte des Vermischens einer Guanidin-hältigen Alkoxysilan-Verbindung der nachstehenden allgemeinen Formel (1): 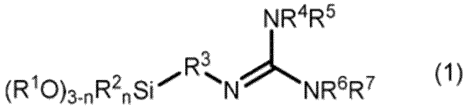
worin R¹ bis R⁷ und n wie oben definiert sind, mit einem Zink-Halogenid der nachstehenden Formel (2):
ZnX₂ (2)
worin X wie oben definiert ist, um zu bewirken, dass Stickstoff-Atome an Guanidin-Stellen auf der Guanidin-hältigen Alkoxysilan-Verbindung über koordinative Bindungen an Zink-Atome auf dem Zink-Halogenid binden und einen Zink-Komplex bilden, der Guanidin-Liganden aufweist; und des anschließenden Vermischens des resultierenden Zink-Komplexes, der Guanidin-Liganden aufweist, mit einem anorganischen Träger umfasst, um kovalente Bindungen zwischen Silicium-Atomen auf dem Zink-Komplex, der Guanidin-Liganden aufweist, und Sauerstoff-Atomen auf einer Oberfläche des anorganischen Trägers zu bilden und um den Zink-Komplex auf dem anorganischen Träger zu immobilisieren.

3. Verfahren zum Synthetisieren eines immobilisierten Zink-Komplexes, der Guanidin-Liganden aufweist, nach Anspruch 1, das die Schritte des Vermischens einer Guanidin-hältigen Alkoxysilan-Verbindung der nachstehenden allgemeinen Formel (1): 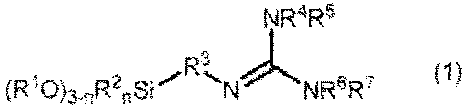
worin R¹ bis R⁷ und n wie oben definiert sind, mit einem anorganischen Träger, um kovalente Bindungen zwischen Sauerstoff-Atomen, die auf einer Oberfläche des anorganischen Trägers vorhanden sind, und Silicium-Atomen auf der Guanidin-hältigen Alkoxysilan-Verbindung zu bilden und um die Guanidin-hältige Alkoxysilan-Verbindung auf dem anorganischen Träger zu immobilisieren, des Erzeugens eines organisch-anorganischen Verbundmaterials; und des anschließenden Vermischens des organisch-anorganischen Verbundmaterials mit einem Zink-Halogenid der nachstehenden allgemeinen Formel (2):
ZnX₂ (2)
worin X wie oben definiert ist, umfasst, um zu bewirken, dass Stickstoff-Atome an Guanidin-Stellen, die in dem organisch-anorganischen Verbundmaterial enthalten sind, über koordinative Bindungen an Zink-Atome auf dem Zink-Halogenid binden, wodurch ein Zink-Komplex, der Guanidin-Liganden aufweist, erzeugt wird.

4. Verfahren zum Synthetisieren einer zyklischen Carbonat-Verbindung der nachstehenden allgemeinen Formel (6): 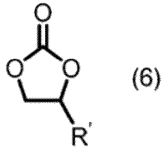
worin R' ein Wasserstoffatom, eine einwertige Kohlenwasserstoffgruppe mit 1 bis 18 Kohlenstoffatomen ist, auf der gegebenenfalls manche oder alle der Wasserstoffatome durch andere Substituenten ersetzt sind, die aus der aus einer Alkoxygruppe mit 1 bis 3 Kohlenstoffatomen, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, wobei die einwertige Kohlenwasserstoffgruppe gegebenenfalls ein Heteroatom, eine Gruppe der nachstehenden allgemeinen Formel (4):
-R⁸-SiR⁹ₘ(OR¹⁰)₃₋ₘ (4)
worin R⁸ eine unsubstituierte zweiwertige Kohlenwasserstoffgruppe mit 1 bis 10 Kohlenstoffatomen ist, die gegebenenfalls ein Heteroatom enthält, R⁹ und R¹⁰ jeweils unabhängig voneinander eine einwertige Kohlenwasserstoffgruppe mit 1 bis 10 Kohlenstoffatomen sind, auf der gegebenenfalls manche oder alle der Wasserstoffatome durch andere Substituenten ersetzt sind, die aus der aus einer Alkoxygruppe mit 1 bis 3 Kohlenstoffatomen, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, und m eine ganze Zahl von 0 bis 3 ist, oder eine Gruppe der nachstehenden allgemeinen Formel (7) umfasst: 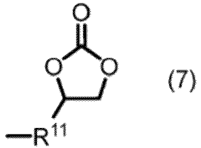
worin R¹¹ eine zweiwertige Kohlenwasserstoffgruppe mit 1 bis 20 Kohlenstoffatomen ist, auf der gegebenenfalls manche oder alle der Wasserstoffatome durch andere Substituenten ersetzt sind, die aus der aus einer Alkoxygruppe mit 1 bis 3 Substituenten, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, wobei die zweiwertige Kohlenwasserstoffgruppe gegebenenfalls ein Heteroatom umfasst,
wobei das Verfahren den Schritt des Bewirkens einer Cycloaddtions-Reaktion zwischen einem Epoxid der nachstehenden allgemeinen Formel (3): 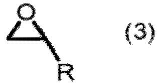
worin R ein Wasserstoffatom, eine einwertige Kohlenwasserstoffgruppe mit 1 bis 18 Kohlenstoffatomen, auf der gegebenenfalls manche oder alle der Wasserstoffatome durch andere Substituenten ersetzt sind, die aus der aus einer Alkoxygruppe mit 1 bis 3 Kohlenstoffatomen, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, wobei die einwertige Kohlenwasserstoffgruppe gegebenenfalls ein Heteroatom, eine Gruppe der nachstehenden allgemeinen Formel (4):
-R⁸-SiR⁹ₘ(OR¹⁰)₃₋ₘ (4)
worin R⁸ bis R¹⁰ und m wie oben definiert sind, oder eine Gruppe der nachstehenden allgemeinen Formel (5) umfasst: 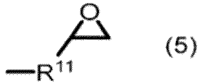
worin R¹¹ eine zweiwertige Kohlenwasserstoffgruppe mit 1 bis 20 Kohlenstoffatomen ist, auf der gegebenenfalls manche oder alle der Wasserstoffatome durch andere Substituenten ersetzt sind, die aus der aus einer Alkoxygruppe mit 1 bis 3 Kohlenstoffatomen, einem Halogenatom, einer Arylgruppe mit 6 bis 10 Kohlenstoffatomen, einer Aralkylgruppe mit 7 bis 10 Kohlenstoffatomen, einer Cyanogruppe, einer Aminogruppe, einer Estergruppe, einer Ethergruppe, einer Carbonylgruppe, einer Acylgruppe und Sulfidgruppen bestehenden Gruppe ausgewählt sind, wobei die zweiwertige Kohlenwasserstoffgruppe gegebenenfalls ein Heteroatom umfasst,
und von Kohlendioxid in Gegenwart eines Katalysators umfasst, wobei ein immobilisierter Zink-Komplex, der Guanidin-Liganden aufweist, nach Anspruch 1 als Katalysator eingesetzt wird.

## Revendications

1. Complexe de zinc immobilisé ayant des ligands guanidine, comprenant au moins un composé alcoxysilane contenant une guanidine de formule générale (1) ci-dessous 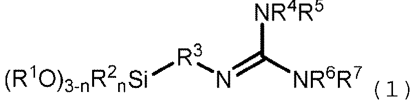
dans laquelle R¹ et R² sont chacun indépendamment un groupe hydrocarboné monovalent de 1 à 10 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure ; R³ est un groupe hydrocarboné divalent non substitué de 1 à 10 atomes de carbone qui peut comprendre un hétéroatome ; R⁴ à R⁷ sont chacun indépendamment un atome d'hydrogène ou un groupe hydrocarboné monovalent non substitué de 1 à 10 atomes de carbone, et R⁴ et R⁵*,* R⁶ et R⁷, et R⁴ et R⁶ peuvent chacun se lier mutuellement et former un cycle avec l'atome ou les atomes d'azote auxquels ils sont liés ; et n est un entier de 0 à 2, un halogénure de zinc de formule générale (2) ci-dessous
ZnX₂ (2)
dans laquelle X est un atome d'halogène et un support inorganique,
dans lequel les atomes de silicium sur le complexe de zinc ayant des ligands guanidine, où des atomes d'azote aux sites provenant de la guanidine sur le composé alcoxysilane contenant une guanidine sont attachés à des atomes de zinc de l'halogénure de zinc par des liaisons de coordination, sont immobilisés sur le support inorganique par des liaisons covalentes avec des atomes d'oxygène à la surface du support inorganique.

2. Procédé de synthèse du complexe de zinc immobilisé ayant des ligands guanidine selon la revendication 1, comprenant les étapes consistant à mélanger un composé alcoxysilane contenant une guanidine de formule générale (1) ci-dessous 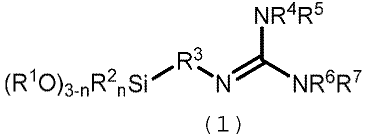
dans laquelle R¹ à R⁷ et n sont tels que définis ci-dessus avec un halogénure de zinc de formule générale (2) ci-dessous
ZnX₂ (2)
dans laquelle X est tel que défini ci-dessus, à faire en sorte que des atomes d'azote aux sites guanidine sur le composé alcoxysilane contenant une guanidine se lient à des atomes de zinc de l'halogénure de zinc par des liaisons de coordination et forment un complexe de zinc ayant des ligands guanidine ; et à mélanger ensuite le complexe de zinc ayant des ligands guanidine obtenu avec un support inorganique pour former des liaisons covalentes entre des atomes de silicium du complexe de zinc ayant des ligands guanidine et des atomes d'oxygène à la surface du support inorganique et immobiliser le complexe de zinc sur le support inorganique.

3. Procédé de synthèse du complexe de zinc immobilisé ayant des ligands guanidine selon la revendication 1, comprenant les étapes consistant à mélanger un composé alcoxysilane contenant une guanidine de formule générale (1) ci-dessous 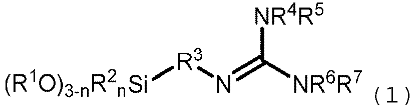
dans laquelle R¹ à R⁷ et n sont tels que définis ci-dessus avec un support inorganique pour former des liaisons covalentes entre des atomes d'oxygène présents à la surface du support inorganique et des atomes de silicium du composé alcoxysilane contenant une guanidine et immobiliser le composé alcoxysilane contenant une guanidine sur le support inorganique, produisant un matériau composite organique-inorganique ; et à mélanger ensuite ce matériau composite organique-inorganique avec un halogénure de zinc de formule générale (2) ci-dessous
ZnX₂ (2)
dans laquelle X est tel que défini ci-dessus, à faire en sorte que des atomes d'azote aux sites guanidine inclus dans le matériau composite organique-inorganique se lient à des atomes de zinc de l'halogénure de zinc par des liaisons de coordination, produisant un complexe de zinc ayant des ligands guanidine.

4. Procédé de synthèse d'un composé carbonate cyclique de formule générale (6) ci-dessous 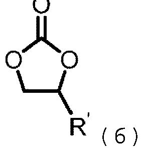
dans laquelle R' est un atome d'hydrogène, un groupe hydrocarboné monovalent de 1 à 18 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure, et le groupe hydrocarboné monovalent peut comprendre un hétéroatome, un groupe de formule générale (4) ci-dessous
-R⁸-SiR⁹ₘ(OR¹⁰)₃₋ₘ (4)
dans laquelle R⁸ est un groupe hydrocarboné divalent non substitué de 1 à 10 atomes de carbone qui peut comprendre un hétéroatome, R⁹ et R¹⁰ sont chacun indépendamment un groupe hydrocarboné monovalent de 1 à 10 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure, et m est un entier de 0 à 3 ou un groupe de formule générale (7) ci-dessous 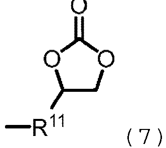
dans laquelle R¹¹ est un groupe hydrocarboné divalent de 1 à 20 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure, et le groupe hydrocarboné divalent peut comprendre un hétéroatome, lequel procédé comprend l'étape de réaliser une réaction de cycloaddition entre un époxyde de formule générale (3) ci-dessous 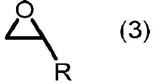
dans laquelle R est un atome d'hydrogène, un groupe hydrocarboné monovalent de 1 à 18 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure, et le groupe hydrocarboné monovalent peut comprendre un hétéroatome, un groupe de formule générale (4) ci-dessous
-R⁸-SiR⁹ₘ(OR¹⁰)₃₋ₘ (4)
dans laquelle R⁸ à R¹⁰ et m sont tels que définis ci-dessus ou un groupe de formule générale (5) ci-dessous 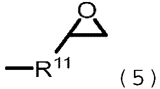 dans laquelle R¹¹ est un groupe hydrocarboné divalent de 1 à 20 atomes de carbone dont tout ou partie des atomes d'hydrogène peuvent être remplacés par d'autres substituants choisis dans le groupe constitué par un groupe alcoxy de 1 à 3 atomes de carbone, un atome d'halogène, un groupe aryle de 6 à 10 atomes de carbone, un groupe aralkyle de 7 à 10 atomes de carbone, un groupe cyano, un groupe amino, un groupe ester, un groupe éther, un groupe carbonyle, un groupe acyle et des groupes sulfure et le groupe hydrocarboné divalent peut comprendre un hétéroatome et du dioxyde de carbone en présence d'un catalyseur, dans lequel le complexe de zinc immobilisé ayant des ligands guanidine de la revendication 1 est utilisé comme catalyseur.
